Europäisches Patentamt

European Patent Office

Office européen des brevets

(19)

(11) Veröffentlichungsnummer : **0 089 601**
**B1**

(12) **EUROPÄISCHE PATENTSCHRIFT**

(45) Veröffentlichungstag der Patentschrift :
02.01.86

(21) Anmeldenummer : 83102533.3

(22) Anmeldetag : 15.03.83

(51) Int. Cl.⁴ : **A 61 F 13/06**

(54) **Patellarsehnen-Bandage.**

(30) Priorität : **19.03.82 DE 3210060**

(43) Veröffentlichungstag der Anmeldung :
**28.09.83 Patentblatt 83/39**

(45) Bekanntmachung des Hinweises auf die Patenterteilung : **02.01.86 Patentblatt 86/01**

(84) Benannte Vertragsstaaten :
**AT BE CH FR GB IT LI LU NL SE**

(56) Entgegenhaltungen :
**DE-A- 3 016 426**

(73) Patentinhaber : **Hildebrandt, Hans-Dietrich, Dr. med.**
**Hohlesteinweg 16**
**D-3501 Ahnatal (DE)**

(72) Erfinder : **Hildebrandt, Hans-Dietrich, Dr. med.**
**Hohlesteinweg 16**
**D-3501 Ahnatal (DE)**

(74) Vertreter : **Freiherr von Schorlemer, Reinfried**
**Brüder-Grimm-Platz 4**
**D-3500 Kassel (DE)**

EP 0 089 601 B1

## Beschreibung

Die Erfindung betrifft eine Patellarsehnen-Bandage der im Oberbegriff des Anspruchs 1 definierten Gattung.

Patellarsehnen-Bandagen werden bei degenerativen Knorpelveränderungen der Kniescheibe (Chondropathia patellae) oder bei durch retropatellare Arthrosen bedingten Schmerzen verordnet. Es handelt sich hierbei um Verschleißerscheinungen unterschiedlichen Ausmasses im Bereich der Kniescheibenrückseite, insbesondere um eine Knorpelerweichung bis zum teilweisen Verlust der Knorpelfläche, bzw. um Verschleißerscheinungen im femuro patellae — Gelenk insgesamt mit Knorpelschäden auch im Bereich der Gleitfläche des Oberschenkelknochens für die Kniescheibe (Patella) und Knochenneubildungen im Sinne von Osteophyten. Mit dem Anlegen der eingangs bezeichneten Bandagen wird versucht, durch Druck auf die von der unteren Kniescheibenspitze bis zur Tuberositas tibiae (rauher Höcher am oberen Ende des Schienbeins) verlaufende Patellarsehne Einfluß auf die Anpreßfläche und den Anpreßdruck der Kniescheibenrückfläche zu nehmen.

Im einzelnen ist nachgewiesen, daß durch den Druck der Bandage auf die Patellarsehne die Kniescheibe insbesondere in ihrem oberen Teil leicht ventral abgekippt und dadurch der Anpreßdruck der Kniescheibe auf den Oberschenkelknochen vermindert wird. Darüberhinaus werden durch Druck der Bandage auf die Patellarsehne reflektorische Vorgänge diskutiert, die zu einer Verminderung des Tonus der Quadricepsmuskulatur führen und somit zusätzlich den Anpreßdruck der Kniescheibe vermindern.

Die bisher bekannten Patellarsehnen-Bandagen weisen verschiedene Unzulänglichkeiten auf, die insbesondere auf einer ungünstigen Ausbildung ihres Funktionsabschnitts beruhen. Bei einer bekannten Ausführungsform besteht der Funktionsabschnitt beispielsweise aus einem etwa 20 Millimeter breiten, flexiblen Band, an das sich ein gepolsterter Verschlußabschnitt anschließt, der beim Tragen der Bandage in der Kniekehle zu liegen kommt. Ein derartiger Funktionsabschnitt rutscht beim Tragen der Bandage leicht bis oder sogar über die Tuberositas tibiae und übt dann keinerlei Wirkung mehr aus. Selbst ein sehr festes Anziehen der Bandage kann nicht sicherstellen, daß der Funktionsabschnitt im Bereich der Patellarsehne angeordnet bleibt. Abgesehen davon hätte ein zu festes Anziehen der Bandage einen starken Druck des Verschlußteils und dadurch unangenehme Begleiterscheinungen im Bereich der Kniekehle zu Folge, was vermieden werden sollte. Ähnliche Probleme ergeben sich bei Anwendung anderer bekannter Patellarsehnen-Bandagen, die einen etwa 13 Millimeter breiten, im Querschnitt ovalen bis kreisförmigen Funktionsabschnitt aufweisen. Eine bekannte Kniegelenkbandage ist in DE-A-3 016 426 dargelegt. Der Erfindung liegt die Aufgabe zugrunde, die Patellarsehnen-Bandage der eingangs bezeichneten Gattung so auszubilden, daß sie beim Tragen nicht oder nur so wenig rutscht, daß der erwünschte, ständige und gleichmäßige Druck auf die Patellarsehne auch bei längerem Gebrauch nicht verloren gehen kann.

Zur Lösung dieser Aufgabe sind die kennzeichnenden Merkmale des Anspruchs 1 vorgesehen.

Weitere vorteilhafte Merkmale der Erfindung ergeben sich aus den abhängigen Ansprüchen.

Die Erfindung bringt den Vorteil mit sich, daß der Funktionsabschnitt schon wegen seiner besonderen Formgebung wenig Neigung zum Rutschen hat. Verstärkt wird die geringe Rutschneigung dadurch, daß sich die Unterkante des Funktionsabschnitts wegen der leicht konischen, dem Beinabschnitt unterhalb der Kniescheibe angepaßten Form und der vergleichsweise großen Breite des Funktionsabschnitts entweder schon beim Anlegen oder nach geringfügigem Verrutschen am oberen Rand der Tuberositas tibiae abstützen kann, die dann ein weiteres Rutschen verhindert. Der Wulst stellt schließlich sicher, daß unabhängig von der im Einzelfall sich einstellenden Lage der Bandage am Bein stets ein gleichmäßiger Druck auf die Patellarsehne ausgeübt wird, ohne daß die Bandage hierzu allzu fest angezogen werden braucht.

Die Erfindung wird nachfolgend in Verbindung mit der beiliegenden Zeichnung an einem Ausführungsbeispiel näher erläutert. Es zeigen :

Figur 1  eine Draufsicht auf die erfindungsgemäße Patellarsehnen-Bandage im abgerollten Zustand ;

Figur 2  einen Schnitt längs der Linie II-II der Fig. 1 ; und

Figur 3  eine Unteransicht der Bandage nach Fig. 1 im Gebrauchszustand.

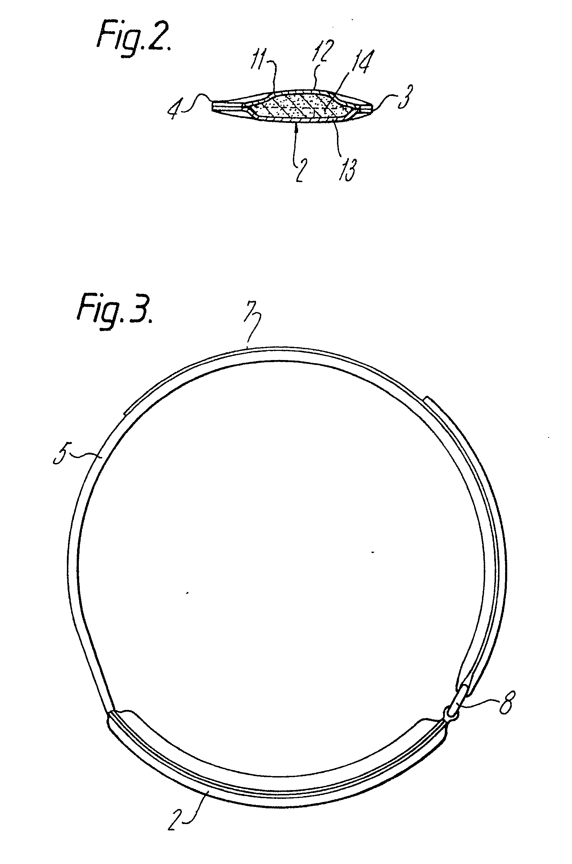

Die erfindungsgemäße Patellarsehnen-Bandage 1 weist einen Funktionsabschnitt 2 auf, der eine bogenförmige Unterkante 3 und eine dazu im wesentlichen parallele Oberkante 4 aufweist. Die Krümmung der beiden Kanten 3 und 4 ist derart gewählt, daß der Funktionsabschnitt im Gebrauchszustand (Fig. 3) eine dem zugehörigen Beinabschnitt unterhalb der Kniescheibe angepaßte, leicht konische Form besitzt. Vorzugsweise verläuft der Funktionsabschnitt 2 längs einer Kegelstumpffläche, so daß er sich beim Tragen dem unterhalb der Kniescheibe befindlichen Beinabschnitt fest und gleichförmig anschmiegen kann.

An den Enden des Funktionsabschnitts 2 sind Verschlußelemente vorgesehen, die beispielsweise aus bandförmigen Streifen 5 und 6 bestehen, von denen der eine Schichten 7 zur Bildung eines Klettenverschlusses aufweist, während der andere an seinem freien Ende mit einem Ring bzw. einer Öse 8 od. dgl. versehen ist, durch die der Streifen 5 durchgezogen und dann auf sich selbst zurückgefaltet wird.

Die senkrecht zu den Kanten 3 bzw. 4 gemessene Breite b des Funktionsabschnitts 2 beträgt etwa 35 Millimeter und ist daher größer als die durchschnittliche Breite des Zwischenraums zwischen Kniescheibe und Tuberositas tibiae. Um bei dieser Breite der Bandage eine Irritation der unteren Kniescheibenspitze durch Druck zu vermeiden, die häufig infolge einer sekundären Entzündung des Ansatzes der Patellarsehne schmerzhaft gereizt ist (Patellarspitzensyndrom), weist die Bandage 1 in einem dem Zwischenraum zwischen Kniescheibe und Tuberositas tibiae zugeordneten Bereich 9 eine an die Oberkante 4 grenzende Aussparung 10 auf, in welcher die Kniescheibenspitze beim Anlegen der Bandage 1 zu liegen kommt, ohne vom Funktionsabschnitt 2 berührt zu werden. Dadurch ist die Bandage 1 im Bereich 9 nur noch so breit, wie dem Abstand zwischen Kniescheibenspitze und Tuberositas tibiae entspricht, d. h. vorzugsweise etwa 30 Millimeter breit.

Auf seiner Innenseite weist der Funktionsabschnitt 2 einen Wulst 11 auf, der sich vorzugsweise nur über einen mittleren Teil des Funktionsabschnitts 2 und etwa parallel zwischen den Kanten 3 und 4 erstreckt. Dieser Wulst 11 kommt beim Tragen der Bandage auf der Patellarsehne zu liegen und übt Druck auf diese auf. Die parallel zu den Kanten 3, 4 gemessene Länge des Wulstes 11 sollte wenigstens der Länge des Bereichs 9 entsprechen, doch kann der Wulst 11 auch genau so lang wie der Funktionsabschnitt 2 sein.

Nach dem Anlegen der Bandage befindet sich die Unterkante 3 des Funktionsabschnitts 2 aufgrund dessen relativ großer Breite dicht oberhalb des oberen Randes der Tuberositas tibiae, während der Wulst 11 auf einem mittleren Teil der Patellarsehne aufliegt. Der Funktionsabschnitt 2 kann daher, sofern die beschriebene Form der Bandage allein nicht ausreicht, um ein Rutschen während des Gebrauchs völlig zu verhindern, allenfalls so weit rutschen, bis ich seine Unterkante 3 auf den oberen Rand der Tuberositas tibiae auflegt und konsolenartig auf diesem abstützt. Die nach unten leicht konische Verjüngung des Funktionsabschnitts 2 fördert diesen Effekt noch. Daher ist es zweckmäßig, die Höhe des Wulstes 11 und den Abstand zwischen dem höchsten Teil 12 des Wulstes 11 und der Unterkante 3 so zu wählen, daß der Wulst 11 auch bei Abstützung der Unterkante 3 auf dem oberen Rand der Tuberositas tibiae noch ausreichend Druck auf einen mittleren Abschnitt der Patellarsehne ausübt. Dadurch ist sichergestellt, daß die erwünschte Druckwirkung unabhängig von der Lage ausgeübt wird, welche der Funktionsabschnitt 2 letzlich einnimmt. Alternativ kann die Anordnung so getroffen sein, daß die Unterkante 3 des Funktionsabschnitts 2 schon beim Anlegen der Bandage auf dem oberen Rand der Tuberositas tibiae zu liegen kommt.

Der Funktionsabschnitt 2 braucht nicht exakt wie ein Kegelstumpfmantel geformt sein, weil bereits die durch den bogenförmigen Verlauf von Unterkante 3 und Oberkante 4 erzielte Form ein Rutschen der Bandage weitgehend ausschließt, auch wenn die Kanten 3 und 4 nicht parallel zueinander verlaufen und die Krümmungsradien unter Berücksichtigung der Anatomie des Beins ausreichend groß gewählt sind. Bei einer praktischen Ausführungsform beträgt die Länge der Unterkante 3 etwa 8 cm, die Länge der Oberkante 4 etwa 8,5 cm und die Breite b außerhalb der Aussparung 9 etwa 3,5 cm, im Bereich der Aussparung 9 dagegen etwa 3,0 cm. Die Streifen 5 und 6 schließen sich an den beiden Enden des Funktionsabschnitts 2 etwa tangential an und besitzen vorzugsweise etwa dieselbe Breite wie der Funktionsabschnitt, damit eine gute Druckverteilung erzielt wird und dadurch unangenehme Begleiterscheinungen durch Druck des Verschlußteils in der Kniekehle vermieden werden.

Obwohl eine Bandage 1 mit den angegebenen Dimensionen bei geeigneter Länge der Streifen 5 und 6 praktisch für Menschen jeder Größe brauchbar ist, kann die erfindungsgemäße Bandage in zwei oder mehr Größen hergestellt und geliefert werden, damit auch bei besonders stark oder schwach entwickelten Beinen der erwünschte feste Sitz gewährleistet ist bzw. auch bei sehr kleinen oder sehr großen Menschen der Wulst 11 jedenfalls so lange Druck auf die Patellarsehne ausübt, wie die Unterkante 3 noch auf der Tuberositas tibiae abgestützt ist. Außerdem könnte vorgesehen sein, den Kanten 3 und 4 unterschiedliche Krümmungsradien zu geben.

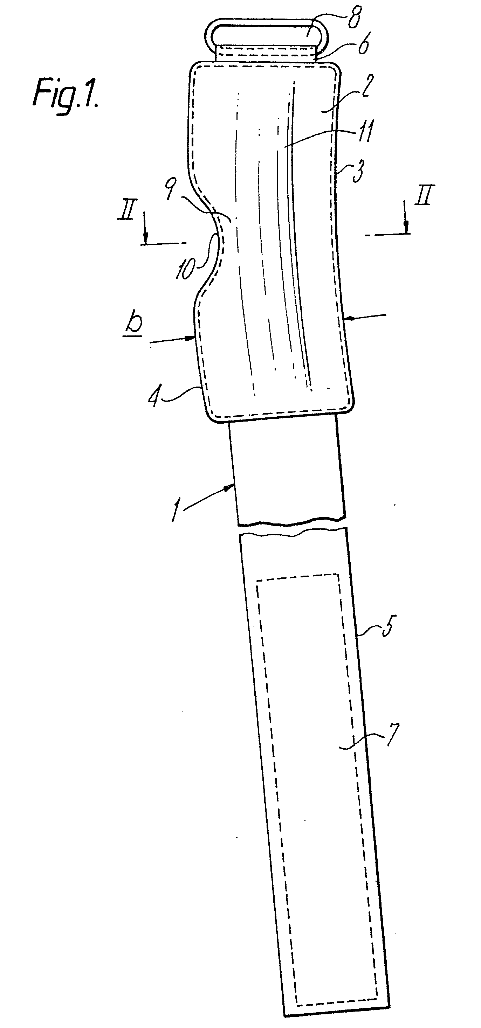

Als Materialien für den Funktionsabschnitt eignen sich beispielsweise Leder oder Kunstleder. Die beiden Streifen 5 und 6 können dabei entweder am Funktionsabschnitt beispielsweise durch Nähen befestigt sein oder die Endabschnitte eines durchgehenden Bandes bilden, dessen Mittelteil mit einem den Funktionsabschnitt bildenden Materialabschnitt derart umwickelt und beispielsweise vernäht ist, daß sich die Form nach Fig. 1 ergibt. Auch andere Ausführungsformen sind denkbar. Die Streifen 5 und 6 bestehen vorzugsweise aus einem normalen Gurtband. Der Wulst 11 wird vorzugsweise dadurch gebildet, daß der Funktionsabschnitt 2 entsprechend Fig. 2 einen Außenmantel 13 aus weichem Leder oder Kunstleder erhält und der innere Hohlraum dieses Außenmantels mit einem Füllstück 14 aus einem weichen, jedoch formbeständigen Material, z. B. einem Schaumstoff, ausgefüllt wird, das an den den Kanten 3 und 4 zugewandten Enden vergleichsweise dünn ist, in seinem mittleren Teil eine konvexe Querschnittsverdickung besitzt und aus einer Schaumstoffplatte entsprechend ausgeschnitten oder aus mehreren Schaumstofflagen zusammengelegt ist. Zur Lagefixierung können die in Berührung befindlichen Teile des Außenmantels 13 und des Füllstücks 14 miteinander verklebt oder vernäht sein. An der dem Wulst 11 gegenüber liegenden Breitseite kann außerdem zwischen des Füllstück 14 und den Außenmantel 13 ein Blatt aus einem harten, wenig biegesteifen Material eingelegt sein, um zu vermeiden, daß sich der Wulst 11 an der falschen Breitseite des Funktionsabschnitts 2 ausbildet.

Weiterhin ist es zweckmäßig, den ulst 11 entsprechend Fig. 2 auf einen mittleren, zwischen den Kanten 3 und 4 gelegenen Teil des Funktionsabschnitts zu begrenzen und den übrigen Teil des Funktionsabschnitts mit im wesentlichen planparallelen Breitseiten zu versehen, damit der gewünschte Abstützeffekt nicht durch das Aufliegen des Wulstes 11 auf den unmittelbar an die Tuberositas tibiae grenzenden Teil der Patellarsehne beeinträchtigt wird. Möglich wäre allerdings auch, die Innenseite des Funktionsabschnitts im wesentlichen leicht konvex auszubilden, d. h. den Wulst 11 über die gesamte Breite des Funktionsabschnitts 2 zu erstrecken, oder den Wulst von einem mittleren Teil des Funktionsabschnitts bis zu dessen Oberkante 4 durchlaufen zu lassen.

Anstelle der Aussparung 10 kann vorgesehen sein, dem Funktionsabschnitt durchgehend eine Breite zu geben, die der Breite im Bereich 9 entspricht, d. h. etwa 30 Millimeter beträgt. In diesem Fall dürfte es jedoch erforderlich sein, für größere oder kleinere Personen etwas breitere oder schmalere Funktionsabschnitte vorzusehen, um den unterschiedlichen Abständen zwischen der Kniescheibenspitze und der Tuberositas tibiae gerecht zu werden.

Als Verschlußelemente können andere als die dargestellten, einen Klettenverschluß bildenden Verschlußelemente vorgesehen werden, um die Bandage am Bein zu fixieren. Der freie Bügel des Rings 8 kann beispielsweise eine Rolle tragen, die das Festziehen des in den Ring 8 eingelegten Streifens 5 beim Anlegen der Bandage erleichtert.

**Patentansprüche**

1. Patellarsehnen-Bandage, bestehend aus einem unterhalb der Kniescheibe um das Bein zu legenden Band, das einen auf die Patellarsehne einwirkenden Funktionsabschnitt (2) und an diesem befestigte Verschlußelemente (7, 8) aufweist, dadurch gekennzeichnet, daß der Funktionsabschnitt (2) einen dem Zwischenraum zwischen Kniescheibe und Tuberositas tibiae zugeordneten Bereich (9) mit einer im wesentlichen der Breite dieses Zwischenraums entsprechenden Breite, eine dem zugehörigen Beinabschnitt angepaßte Form und einen auf seiner Innenseite gelegenen, zum Druck auf die Patellarsehne bestimmten Wulst (11) aufweist.

2. Patellarsehen-Bandage nach Anspruch 1, dadurch gekennzeichnet, daß der Funktionsabschnitt (2) längs eines Kegelstumpfmantels verläuft.

3. Patellarsehnen-Bandage nach Anspruch 1 oder 2, dadurch gekennzeichnet, daß sich der Wulst (11) lediglich über einen mittleren Bereich des Funktionsabschnitts (2) erstreckt.

4. Patellarsehnen-Bandage nach einem der Ansprüche 1 bis 3, dadurch gekennzeichnet, daß der höchste Teil (12) des Wulstes (11) einen solchen Abstand von der Unterkante (3) des Funktionsabschnitts (2) besitzt, daß er bei Abstützung dieser Unterkante (3) auf dem oberen Rand der Tuberositas tibiae auf einem mittleren Abschnitt der Patellarsehne zu liegen kommt.

5. Patellarsehnen-Bandage nach einem der Ansprüche 1 bis 4, dadurch gekennzeichnet, daß sie aus einem durchgehenden Band besteht, das mit einem den Funktionsabschnitt (2) bildenden Element umwickelt ist.

6. Patellarsehnen-Bandage nach einem der Ansprüche 1 bis 5, dadurch gekennzeichnet, daß die Oberfläche des Funktionsabschnitts (2) aus einem rutschfesten Material besteht.

7. Patellarsehnen-Bandage nach Anspruch 6, dadurch gekennzeichnet, daß der Außenmantel des Funktionsabschnitts (2) aus Kunstleder besteht.

8. Patellarsehnen-Bandage nach einem der Ansprüche 1 bis 7, dadurch gekennzeichnet, daß der Funktionsabschnitt (2) eine größere Breite besitzt, als dem Zwischenraum zwischen Kniescheibe und Tuberositas tibiae entspricht, und in dem dem Zwischenraum zwischen Kniescheibe und Tuberositas tibiae zugeordneten Bereich (9) eine an seine Oberkante (4) grenzende, zur Aufnahme der Kniescheibenspitze bestimmte Aussparung (10) aufweist.

**Claims**

1. Patellar ligament bandage consisting of a band to be placed around the leg below the patella, the band having a functional portion (2) acting on the patellar ligament and closure elements (7, 8) attached to the functional portion, characterised in that the functional portion (2) has a zone (9) associated with the interval between the patella and the Tuberositas tibiae and which has a width corresponding substantially to the width of this interval, a shape adapted to the associated part of the leg, and a bulge (11) located on its inner side and intended for pressure on the patellar ligament.

2. Patellar ligament bandage according to claim 1, characterised in that the functional portion (2) extends along a frustoconical surface.

3. Patellar ligament bandage according to claim 1 or 2, characterised in that the bulge (11) extends solely over a middle zone of the functional portion (2).

4. Patellar ligament bandage according to any one of claims 1 to 3, characterised in that the highest part (12) of the bulge (11) is at such a distance from the lower edge (3) of the functional portion (2) that when this lower edge (3) is supported on the upper margin of the Tuberositas tibiae this part comes to lie on a middle portion of the patellar ligament.

5. Patellar ligament bandage according to any one of claims 1 to 4, characterised in that it consists of a continuous band which is enveloped by an element forming the functional portion (2).

6. Patellar ligament bandage according to any one of claims 1 to 5, characterised in that the surface of the functional portion (2) consists of a slip-proof material.

7. Patellar ligament bandage according to claim 6, characterised in that the outer shell of the functional portion (2) consists of imitation leather.

8. Patellar ligament bandage according to any one of claims 1 to 7, characterised in that the functional portion (2) has a width greater than that corresponding to the interval between the patella and the Tuberositas tibiae and, in the zone (9) associated with the interval between the patella and the Tuberositas tibiae, has a recess (10) bordering its upper edge (4) which is intended to accommodate the tip of the patella.

**Revendications**

1. Bandage pour ligament rotulien, constitué par une bande destinée à être placée autour de la jambe en dessous de la rotule et comportant une section fonctionnelle (2) agissant sur le ligament rotulien et des éléments de fermeture (7, 8) fixés à cette dernière, caractérisé en ce que la section fonctionnelle (2) comporte une zone (9) associée à l'espace intermédiaire entre la rotule et la tubérosité tibiale, dont la largeur correspond sensiblement à la largeur de cet espace intermédiaire, une forme adaptée à la portion de jambe associée et un bourrelet (11) placé sur son côté intérieur et destiné à exercer une pression sur le ligament rotulien.

2. Bandage pour ligament rotulien selon la revendication 1, caractérisé en ce que la section fonctionnelle (2) s'étend le long d'une enveloppe tronconique.

3. Bandage pour ligament rotulien selon la revendication 1 ou 2, caractérisé en ce que le bourrelet (11) s'étend seulement sur une zone médiane de la section fonctionnelle (2).

4. Bandage pour ligament rotulien selon l'une des revendications 1 à 3, caractérisé en ce que la partie la plus élevée (12) du bourrelet (11) est à une distance du bord inférieur (3) de la section fonctionnelle (2) telle qu'elle vient s'appliquer sur une portion médiane du ligament rotulien, le bord inférieur (3) s'appuyant sur le bord supérieur de la tubérosité tibiale.

5. Bandage pour ligament rotulien selon l'une des revendications 1 à 4, caractérisé en ce qu'il est constitué par une bande continue par laquelle est enroulé un élément constituant la section fonctionnelle (2).

6. Bandage pour ligament rotulien selon l'une des revendications 1 à 5, caractérisé en ce que la surface de la section fonctionnelle (2) est en un matériau qui ne peut pas glisser.

7. Bandage pour ligament rotulien selon la revendication 6, caractérisé en ce que l'enveloppe extérieure de la section fonctionnelle (2) est en cuir synthétique.

8. Bandage pour ligament rotulien selon l'une des revendications 1 à 7, caractérisé en ce que la section fonctionnelle (2) a une largeur supérieure à ce qui correspond à l'espace intermédiaire entre la rotule et la tubérosité tibiale et comporte dans la zone (9) associée à l'espace intermédiaire entre la rotule et la tubérosité tibiale un évidement (10) adjacent à son bord supérieur (4) destiné à recevoir la pointe de la rotule.

Fig.1.

**Fig.2.**

**Fig.3.**